# EUROPÄISCHE PATENTSCHRIFT

(11) **EP 2 900 186 B1**
(45) Veröffentlichungstag und Bekanntmachung des Hinweises auf die Patenterteilung: **16.11.2016**
(21) Anmeldenummer: 13785366.9
(22) Anmeldetag: 30.09.2013
(51) Int. Cl.: A61F 9/007, A61F 9/00

(54) **IMPLANTAT ZUR GLAUKOMBEHANDLUNG**
IMPLANT FOR TREATING GLAUCOMA
IMPLANT POUR LE TRAITEMENT DU GLAUCOME

(30) Priorität: 28.09.2012 WO PCT/IB2012/001937
(43) Veröffentlichungstag der Anmeldung: 05.08.2015
(73) Patentinhaber: Doci Innovations GmbH, 23730 Sierksdorf (DE)
(72) Erfinder: DOCI, Violeta, 22049 Hamburg (DE)
(74) Vertreter: Simandi, Claus
(86) Internationale Anmeldenummer: PCT/EP2013/070367
(87) Internationale Veröffentlichungsnummer: WO 2014/049174

(56) Entgegenhaltungen:
- US-A1- 2004 193 262
- US-A1- 2009 082 863
- US-A1- 2011 224 597

## Beschreibung

### Gebiet der Erfindung

Die vorliegende Erfindung betrifft ein Implantat zur Minderung des Augeninnendrucks. Dieses Implantat kann insbesondere zur Prophylaxe und Behandlung bei sich abzeichnenden oder auftretenden Glaukomen eingesetzt werden.

### Hintergrund der Erfindung

Glaukome (auch Grüner Star genannt) sind eine weitverbreitete Augenkrankheit. Die mit dieser Krankheit verbundene Schädigung des Sehnervs entsteht durch einen Überdruck im Auge. Dieser Überdruck kann operativ gemindert werden. Es existiert eine Vielzahl von Ansätzen zur Therapie von Glaukomen. Eine mögliche Behandlung ist die Implantation einschlägiger Implantate, welche auch als "Stents" bezeichnet werden.

Die US-Patentanmeldung US 2007/0118147 offenbart ein Implantat, welches in den Schlemm'schen Kanal eingesetzt werden soll. Dieses Implantat weist eine Art Röhrensystem auf. Eine erste Röhre weist eine Einlassöffnung auf. Diese Röhre ist über ein Verbindungsstück mit einer Querröhre verbunden. Diese Querröhre verfügt über mehrere Auslässe. Ihre Aufgabe ist es, Flüssigkeit abzugeben.

Es fällt bei diesem Implantat allerdings nachteilig auf, dass es insgesamt recht voluminös und auch raumgreifend ist, also wesentliche Ausdehnung in mehreren Raumrichtungen hat. Insofern ist das Einsetzen des Implantats für den Operateur nicht ganz risikolos, zumal die richtige Orientierung sehr wichtig erscheint, und nach dem Einsetzen ist mit einer langen Ausheilungszeit zu rechnen.

Die US-Patentanmeldung US 2005/0119636 offenbart ein anderes Implantat, welches ebenfalls in den Schlemm'schen Kanal eingesetzt werden soll. Dieses Implantat weist im Wesentlichen die Form eines länglichen Röhrchens auf. Es gibt also einen Einlass und einen dem Einsatz gegenüberliegenden Auslass.

Die US-Patentanmeldung US 2004/0193262 A1 beschreibt einen Stent, der aus einer Formgedächtnislegierung gefertigt werden kann. Dieser Stent hat die klassische Funktion, nach seinem Einsetzen Gefäße zu weiten. Er kann dazu verschiedene Formen annehmen, beispielsweise eine astartige oder auch eine Spiral-Form. In der Patentanmeldung wird offenbart, dass der Stent in das Trabekelmaschenwerk eingesetzt werden soll und dieses aufweiten soll. Der Stent ist in der Weise dimensioniert, dass er vollständig durch den Schlemm'schen Kanal hindurchragen kann. Es findet vermutlich auch ein verbesserter Abfluss durch den Schlemm'schen Kanal statt. Alleine aufgrund seiner Größe ist jedoch der Stent nicht geeignet, den Schlemm'schen Kanal zu stabilisieren. Vielmehr wird der Schlemm'sche Kanal gepresst oder in seiner Form ungünstig verändert. Solchen eventuellen Nachteile beim Flüssigkeitstransport werden bei dem Stent aus dieser US-Patentanmeldung entgegengewirkt, indem der Stent selbst ein Hohlkörper ist, und so Flüssigkeitsableitung durch den Stent erfolgen kann.

Die vorliegende Erfindung strebt es an, den Stand der Technik zu verbessern. Es soll ein Implantat zum Einsetzen in den Schlemm'schen Kanal angeboten werden, welches geringe Abmessungen hat, sich damit leicht und sicher einsetzen lässt, und eine gut vorhersehbare und kontrollierbare Ableitung von Augenflüssigkeit gewährt.

Dies wird durch ein Implantat nach Anspruch 1 und insbesondere auch durch Implantate erreicht, welche im Sinne der Unteransprüche ausgestaltet sind. Die Vorteile werden ebenfalls durch die Verwendung des Implantats im Sinne des Anspruchs 15 erreicht.

Vorliegend geht es also um ein Implantat für den Schlemm'schen Kanal, d.h. ein Implantat, das zumindest zur teilweisen Implantation in den Schlemm'schen Kanal geeignet ist und das für die teilweise Implantation in den Schlemm'schen Kanal optimiert ist. Die Masse des Implantats sind so gewählt, dass es sich teilweise oder sogar auch vollständig in den Schlemm'schen Kanal einsetzen lässt. Daher soll der entsprechende Querschnitt des Implantats vorzugsweise kleiner sein als der Querschnitt des Schlemm'schen Kanals senkreicht zu seiner Umlaufrichtung oder jedenfalls so klein sein, dass sich das Implantat durch diesen Querschnitt des Schlemm'schen Kanals einsetzen lässt.

Das Implantat soll eine Brücke aufweisen, diese wiederum weist einen ersten Endpunkt, einen zweiten Endpunkt, einen ersten Stützbereich, einen zweiten Stützbereich und ein Verbindungsstück auf. Dabei soll sich die Brücke entlang einer ersten Linie vom ersten Endpunkt über den ersten Stützbereich, das Verbindungsstück und den zweiten Stützbereich zum zweiten Endpunkt erstrecken. Der erste Stützbereich grenzt dabei vorteilhafterweise an den ersten Endpunkt an und der zweite Stützbereich grenzt an den zweiten Endpunkt an. Zumindest das Verbindungsstück kann biegsam ausgeführt werden. Daher muss es sich bei der ersten Linie nicht um eine gerade verlaufende Linie handeln. Die Linie beschreibt aber die Hauptverlaufsrichtung oder Hauptachse der jeweiligen Bauteile. Senkrecht zu dieser Linie ergibt sich somit jeweils eine Querschnittsfläche, welche im Vergleich zur gegenüber der Linie verkippten Querschnittsflächen einen minimalen Querschnitt der Bauteile zeigt.

Entlang dieser ersten Linie hat das Verbindungsstück im Durchschnitt eine erste Querschnittsfläche. Die durchschnittliche Querschnittsfläche lässt sich beispielsweise aus einer Vielzahl von Querschnittsflächen durch Bildung des arithmetischen Mittels berechnen. Die erste Querschnittsfläche beschreibt eine Maßzahl, welche sich beispielsweise in Quadratmillimetern angeben ließe, die diesem arithmetischen Mittel entspricht. Dem ersten Stützbereich kann in gleicher Weise eine durchschnittliche Querschnittsfläche zugeordnet werden, welche hierin als zweite Querschnittsfläche bezeichnet wird. Bei der Feststellung der zweiten Querschnittsfläche ist die Form des ersten Stützbereichs nach der Implantation zugrundezulegen. Wie noch näher ausgeführt werden wird, kann nämlich der erste Stützbereich zum Zwecke der leichteren Implantation in eine andere (im Wesentlichen schlankere) Form überführt werden und der Querschnittsbereich nimmt dann erst nach der Implantation seine endgültige Form an.

Im Sinne der Erfindung soll die zweite Querschnittsfläche um mindestens 50% größer sein als die erste Querschnittsfläche. Es kann zweckmäßig sein, wenn die zweite Querschnittsfläche um mehr als 75%, 100%, 150%, 200%, 300%, 400%, 500% oder mehr größer ist, wobei der Unterschied in der Regel als kleiner 2000% bleiben kann.

Es ist in der Regel zweckmäßig, ein Implantat vorzusehen, bei dem auch der zweite Stützbereich eine deutlich größere Querschnittsfläche aufweist als die erste Querschnittsfläche. Der zweite Stützbereich weist nach der Implantation entlang der ersten Linie im Durchschnitt eine dritte Querschnittsfläche auf. Diese dritte Querschnittsfläche soll um mindestens 50% größer sein als die erste Querschnittsfläche. Vorteilhaft sind im Übrigen auch die prozentualen Größenunterschiede, die oben in Bezug auf die zweite Querschnittsfläche und die erste Querschnittsfläche angegeben wurden.

Die zweite und die dritte Querschnittsfäche sollten etwa 10 000 µm² bis 100 000 µm² groß sein. Dies erlaubt in der Regel die gute Einführung in den Schlemm'schen Kanal.

Bezogen auf die Form, welche der erste Stützbereich, aber auch der zweite Stützbereich zur Implantation einnehmen kann, ist es vorteilhaft, wenn die entsprechenden Querschnittsflächen nicht sehr viel größer sind als die erste Querschnittsfläche. Zweckmäßig ist es, wenn sich der erste Stützbereich zur Implantation in eine Stellung überführen lässt, in der er entlang der ersten Linie im Durchschnitt eine vierte Querschnittsfläche aufweist, welche um nicht mehr als 40% größer ist als die erste Querschnittsfläche. Entsprechend ist es ebenfalls nützlich, wenn der zweite Stützbereich zur Implantation in eine Stellung überführt werden kann, in der er entlang der ersten Linie im Durchschnitt eine fünfte Querschnittsfläche aufweist, welche um nicht mehr als 40% größer ist als die erste Querschnittsfläche. Zweckmäßig kann es sein, wenn die vierte und die fünfte Querschnittsfläche der ersten Querschnittsfläche im Wesentlichen identisch zur ersten Querschnittsfläche sind.

Der Übergang von einer für die Implantation günstige Form des Stützbereiches zu einer nach der Implantation günstigen Form des Stützbereiches kann dadurch erfolgen, dass der erste und/oder der zweite Stützbereich nach Implantation eine Spiral-Form annehmen. Vor der Implantation können der erste und/oder der zweite Stützbereich eine gestreckte Form annehmen. Sie können dabei z.B. eine gestreckte Zylinder-Form annehmen und in ihrer Form die Zylinder-Form des Verbindungsstückes fortsetzen.

Um diesen Übergang zu bewirken, ist es nützlich, wenn der erste und/oder der zweite Stützbereich aus einem Formgedächtnismaterial oder einer Formgedächtnislegierung gefertigt sind. Solche Materialien werden bisweilen auch als Memory-Materialien oder Memory-Legierungen bezeichnet. Die Materialien können aus einer ersten Form in eine zweite, auch durchaus komplexere Form überführt werden, ohne dass die zweite Form der ersten Form irgendwie ähnelt. Dies kann beispielsweise durch Temperaturänderung geschehen. Bei einer typischen Formgedächtnislegierung ist es möglich, dass sie im abgekühlten Zustand eine gestreckte Form einnimmt, und bei Aufwärmung eine vorgegebene Form, beispielsweise eine Spiral-Form einnimmt. Ein in dieser Richtung wirkender thermischer Effekt ist für eine Operation am Menschen besonders günstig. Das Implantat kann vor dem Einsetzen in geeigneter Weise abgekühlt werden, und wird durch Körperwärme bedingt seine zweite Form im Körper schnell annehmen.

Wie noch näher erläutert werden wird, soll das Verbindungsstück des Implantats im Wesentlichen der gebogenen Form des Schlemm'schen Kanals folgen. Dazu ist es vorteilhaft, wenn das Verbindungsstück biegsam ist. Ebenfalls ist vorteilhaft, wenn das Verbindungsstück eine geringe Biegesteifigkeit hat als der erste und/oder der zweite Stützbereich. (D.h. die Durchbiegung eines Abschnitts des Verbindungsstücks in Reaktion auf eine bestimmte Testkraft soll größer sein als Durchbiegung eines gleich langen Abschnitts eines Stützbereiches bei gleicher Testkraft.) Dies soll insbesondere in der Form des Implantats nach Implantation der Fall sein. Nach der Implantation sollen nämlich die Stützbereiche eine feste Verankerung in Abschnitten des Schlemm'schen Kanals sicherstellen, wohingegen das Verbindungsstück ohne großen Widerstand eine gebogene Form einnehmen soll.

Vorteilhaft ist auch, wenn das Verbindungsstück entlang seiner Verlaufsachse nicht dehnbar oder stauchbar ist. In dieser Weise sorgt das Verbindungsstück bei festgelegtem Verlauf dafür, dass der Abstand des ersten und des zweiten Stützbereiches kontant bleibt. Ein Verbindungsstück, das weder dehnbar noch stauchbar ist, wird hierin auch als distanzstabil bezeichnet.

Günstig ist ein insgesamt relativ langes Verbindungsstück. Die Länge des Verbindungsstücks kann also seinen Durchmesser um den Faktor 10 möglicherweise aber auch um einen Faktor von 20 oder 30 übertreffen. Ein Faktor von mehr als 50 ist jedoch nicht erforderlich. Da Verbindungsstück kann vorteilhafterweise einen maximalen Durchmesser von 5 bis 150 µm haben. Zweckmäßigerweise kann das Verbindungsstück aus einem runden Draht mit einem Durchmesser von 5 bis 150 µm oder 10 bis 50 µm gefertigt werden.

Beim Konzept des Implantats nach der vorliegenden Erfindung, muss die Brücke keine Flüssigkeit leiten. Daher ist es zweckmäßig, die Brücke (mindestens aber das Verbindungsstück) ohne Hohlräume, also massiv auszuführen.

Neben der Brücke kann das Implantat noch andere Elemente aufweisen. Dabei kommen insbesondere Flüssigkeitsleitvorrichtungen in Frage. Solche Flüssigkeitsleitvorrichtungen können in der Form von Tuben ausgeführt werden. Ein erfindungsgemäßes Implantat kann also neben der Brücke mindestens einen Tubus aufweisen.

Zweckmäßig ist ein Implantat, welches nach der Implantation eine Stellung einnehmen kann, in der die erste Linie gekrümmt ist und somit in einer ersten Ebene verläuft, wobei das Implantat mindestens einen Tubus aufweist, welcher eine Flüssigkeitsleitung durch diese erste Ebene ermöglicht.

Im Sinne der Erfindung ist es allgemein vorteilhaft, eine Brücke mit Flüssigkeitsleiteinrichtungen auszurüsten, welche eine teilweise zur Ebene der Brücke senkrechte Flüssigkeitsleitung ermöglichen. Diese Tuben können in der üblichen Weise zylinderförmige Rohrabschnitte sein. Es ist aber auch möglich, einen Tubus als Spiraldrahtwicklung vorzusehen. Wenn etwa das Verbindungsstück als dünner Draht ausgeführt wird, so kann dieser Draht spiralförmig gewickelt werden, um eine Flüssigkeitsleiteinrichtung der beschriebenen Art herzustellen. Alternativ können die Tuben beispielsweise dadurch in das Verbindungsstück integriert werden, indem dieses in mehrere Abschnitte unterbrochen wird und einzelne Abschnitte benachbarte Tuben miteinander verbinden. Denkbar ist hierzu u.a. eine Verbindung durch Klebung oder Lötung. Vorteilhaft ist auch die einstückige Aufführung von Flüssigkeitsleiteinrichtungen und Brücke.

Um eine Flüssigkeitsleitung durch die erste Ebene zu ermöglichen, kann der mindestens eine genau oder zumindest im Wesentlichen senkrecht zu der ersten Ebene orientiert sein. Der gewünschte Effekt wird aber auch bei Abweichungen von der Senkrechten erreicht, insbesondere dann, wenn der Winkel zur Senkrechten kleiner als 45° oder kleiner als 30° oder kleiner als 10° ist.

Die Tuben können einen im Vergleich zum Verbindungsstück kleinen Durchmesser haben. Beispielsweise genügt es, wenn der Durchmesser des mindestens einen Tubus kleiner ist als 10% oder auch 5% der Länge des ersten Verbindungsstückes. In dieser Weise lassen sich über die Länge des Verbindungsstückes verteilt, mehrere Tuben unterbringen, beispielsweise zwei, drei, vier oder fünf Tuben. Solche Tuben können aus Metall oder auch Kunststoff gefertigt werden. Die Materialien können ähnlich oder gleich wie die für die Brücke sein.

Im Sinne der vorliegenden Erfindung kann also ein Implantat angeboten werden, welches zur operativen Glaukombehandlung geeignet ist, welchen in den fontal geöffeneten Schlemm'schen Kanal eingesetzt werden kann und welches bogenförmige Ausbuchungen oder Tuben aufweist, welche in Öffnungen der Sammelkanäle für Augenwasser gedrückt werden können. Ein solches Implantat kann spiralförmige Stützbereiche aus einem Formgedächtnismaterial aufweisen.

Die Vorteile des erfindungsgemäßen Implantats, wie auch die Unterschiede zu bekannten Implantaten erschließen sich am leichtesten im Zusammenhang mit den Zeichnungen, insbesondere auch Zeichnungen zur Anatomie des Auges. Daher werden diese Aspekte nachfolgend im Zusammenhang mit den Zeichnungen noch näher besprochen.

In den Abbildungen und in den dazugehörigen Beschreibungen sind Merkmale der Erfindung in Kombination beschrieben. Diese Merkmale können allerdings auch in anderen Kombinationen von einem erfindungsgemäßen Gegenstand umfasst werden. Jedes offenbarte Merkmal ist also auch als in technisch sinnvollen Kombinationen mit anderen Merkmalen offenbart zu betrachten. Die Abbildungen sind teilweise leicht vereinfacht und schematisch.
- Fig. 1: zeigt ein erfindungsgemäßes Implantat in einer Lagerkonfiguration;
- Fig. 2: zeigt ein entsprechendes Implantat in einer Konfiguration unmittelbar vor der Implantation;
- Fig. 3: zeigt ein anderes erfindungsgemäßes Implantat in einer Konfiguration, die der nach der Implantation eingenommenen Stellung weitgehend entspricht;
- Fig. 4: zeigt in einer schematischen perspektivischen Darstellung ein menschliches Auge;
- Fig. 5: zeigt in einer Querschnittsdarstellung einen Bereich des menschlichen Auges;
- Fig. 6: zeigt in einer entsprechenden, aber vergrößerten Querschnittsdarstellung einen Querschnitt durch das Auge nach Einsetzen des Implantats;
- Fig. 7: zeigt in einer Fig. 6 entsprechenden Ansicht einen verschobenen parallelen Querschnitt durch das Auge;
- Fig. 8: zeigt ein einer gegenüber Fig. 6 und Fig. 7 verschobenen Querschnittsansicht einen weiteren Querschnitt durch das Auge.

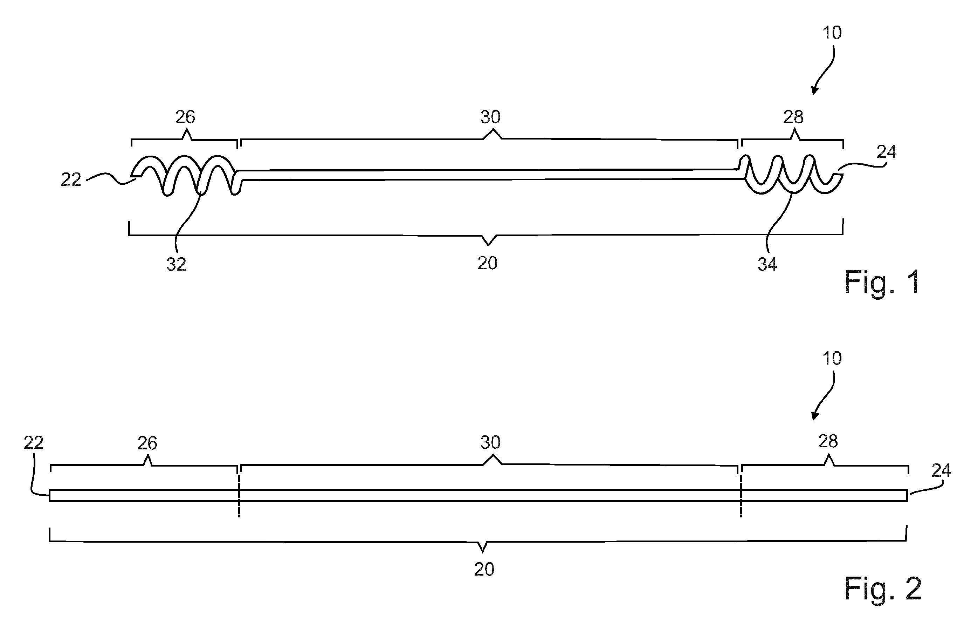

Fig. 1 zeigt ein erfindungsgemäßes Implantat in einer Konfiguration, in der es beispielsweise gelagert werden könnte. Das Implantat besteht in dieser Ausführungsform nur aus der Brücke 20. Die Brücke 20 weist links einen ersten Endpunkt 22 auf. An diesen schließt sich ein erster Stützbereich 26 an. Spiegelbildlich gegenüberliegend findet sich der zweite Endpunkt 24, an den sich der zweite Stützbereich 28 anschließt. Beide Stützbereiche sind durch ein Verbindungsstück miteinander verbunden. Die Stützbereiche sind in dieser Konfiguration spiralförmig, so dass im ersten Stützbereich eine erste Spirale 32 liegt und gegenüber im zweiten Stützbereich 28 eine zweite Spirale 34 liegt.

Fig. 2 zeigt das gleiche Implantat in einer anderen Konfiguration. In diese Konfiguration kann das Implantat unmittelbar vor einer Operation gebracht werden. Es weist keine spiralförmigen Enden auf, sondern der erste Stützbereich 26 und der zweite Stützbereich 28 haben jeweils die Form gestreckter Rundstücke. Sie fügen sich einstückig und formschlüssig an das Verbindungsstück 30 an. Das Implantat kann beispielsweise durch Kühlung in eine solche Form überführt werden.

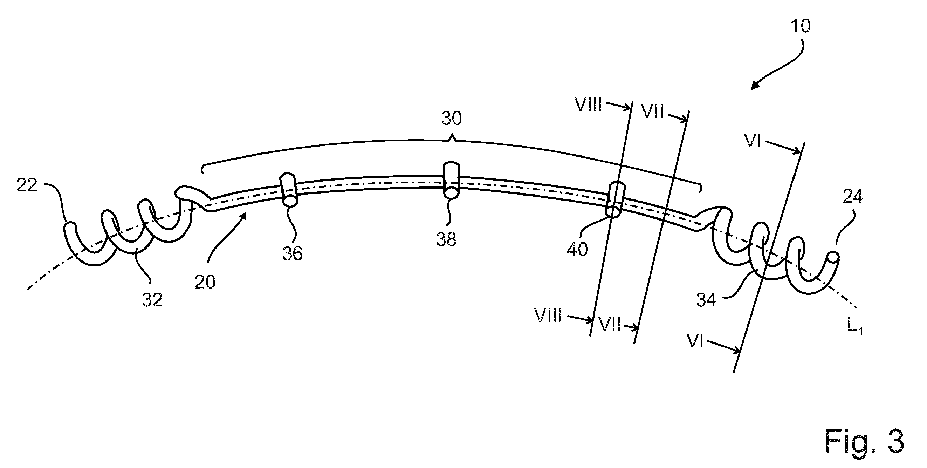

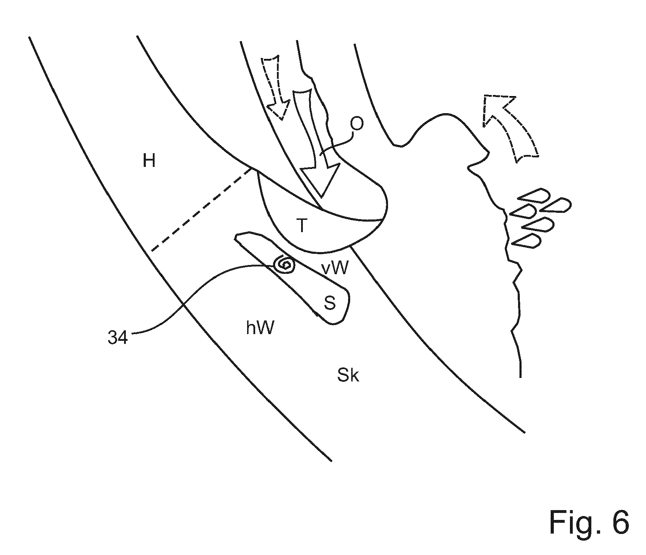

Fig. 3 zeigt ein anderes erfindungsgemäßes Implantat. Dieses weist die schon bekannten Bauteile der Brücke auf. Zusätzlich verfügt dieses Implantat jedoch über drei Tuben, welche äquidistant auf dem Verbindungsstück 30 angeordnet sind. Folglich ist der Abstand zwischen dem ersten Tubus 36 und dem zweiten Tubus 38 genauso groß wie der Abstand zwischen dem zweiten Tubus 38 und dem dritten Tubus 40. Eine äquidistante Anordnung der Tuben ist vorteilhaft, jedoch nicht unbedingt erforderlich. Diese Tuben dienen als Flüssigkeitsleiteinrichtung und ihre genauere Funktion wird nachfolgend bezogen auf die Anatomie des Auges noch näher dargestellt. Die gestrichelten Linien quer zur Verlaufslinie der Brücke 20 geben bezogen auf das Implantat 10 wieder, welche nachfolgend in Fig. 6 bzw. Fig. 7 bzw. Fig. 8 dargestellt sind.

In Fig. 3 ist die Brücke insgesamt in einer gekrümmten Stellung gezeigt. Dabei ist insbesondere das Verbindungsstück 30 gekrümmt. In dieser gekrümmten Stellung passt sich das Implantat dem Verlauf des Schlemm'schen Kanals besonders gut an.

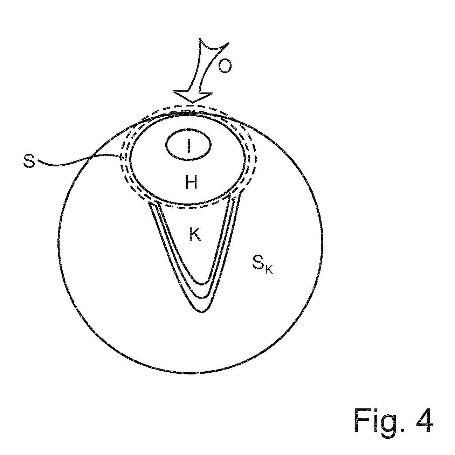

Fig. 4 zeigt eine schematische perspektivische Darstellung des Auges. Oben erkennt man die Hornhaut H und in ihrer Mitte die Iris I. Die Hornhaut wird umgeben von der Sklera Sk.

Um einen besseren Abfluss des Augenwassers zu ermöglichen, muss ggf. ein operativer Zugang zum Schlemm'schen Kanal S gefunden werden. Klassischerweise wurde eine entsprechende Operation von außen ausgeführt. Dabei wurde ein wesentlicher Teil der Sklera Sk geöffnet. Dies konnte in einem konventionellen Operationsfeld K geschehen. Wie in der Zeichnung angedeutet, konnte das Operationsfeld größer oder kleiner gewählt werden, je nachdem über welchen Winkel der Schlemm'sche Kanal zugänglich gemacht werden sollte.

Das Implantat der vorliegenden Erfindung soll jedoch im Zusammenhang mit einer minimal-invasiven Operation verwendet werden. Dies ist ein wichtiger Aspekt, um die Vorteile der vorliegenden Erfindung zu würdigen. Das Implantat ist nämlich in seinen Abmessungen sehr klein gestaltet und eignet sich für eine minimal operative Implantation. Die allgemeine Operationsrichtung ist dabei die Richtung O. Es wird also perpendikular durch die Hornhaut hindurch der Zugang zum Schlemm'schen Kanal S gefunden.

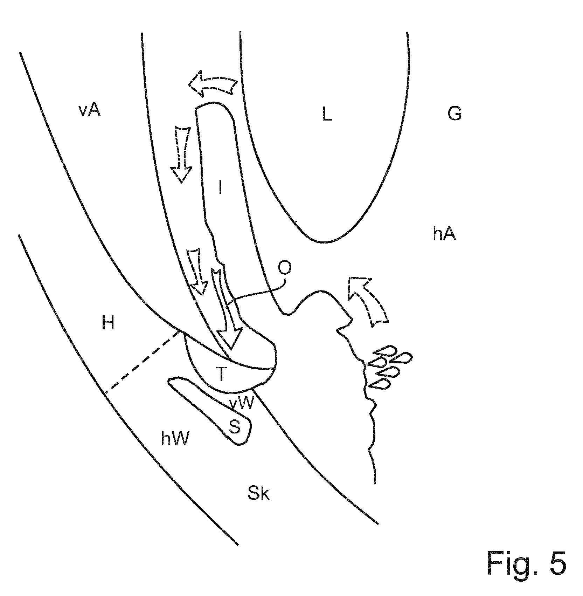

Die zum Verständnis der vorliegenden Erfindung wichtigen Teile des Auges sind auch in der Übersichtsdarstellung der Fig. 5 wiedergegeben. Es handelt sich um eine Querschnittsdarstellung, bei der sich auf der rechten Seite die hintere Augenkammer hA und der Glaskörper G des Auges befinden und sich auf der linken Seite die Hornhaut H befindet. Dazwischen liegen die Linse L und die Iris I.

Augenwasser findet seinen Weg aus Richtung des Glaskörpers an der Linse L vorbei und umfließt die Iris I, so dass das Augenwasser von der hinteren Augenkammer hA in die vordere Augenkammer vA gelangt. Beim gesunden Auge wird es dann durch das Trabekelmaschenwerk weitergeleitet. Das Trabekelmaschenwerk T geht in die vordere Wand vW des Schlemm'schen Kanals S über (wobei dieser Übergang ohne scharfe Grenze ist). Der Schlemm'sche Kanal liegt im Sklera Sk. Er wird nach außen durch eine (aus Sicht des Operateurs) hintere Wand hW begrenzt.

Fig. 6 zeigt einen vergrößerten Ausschnitt aus Fig. 5. In diesem Ausschnitt wird eine Operationstechnik skizziert, welche im Zusammenhang mit der vorliegenden Erfindung eine Rolle spielt. Bei Glaukompatienten ist das Trabekelmaschenwerk T nicht mehr oder nicht mehr hinreichend durchlässig für Augenwasser. Dieses kann daher nicht mehr durch den Schlemm'schen Kanal abgeleitet werden. Es ist in einem minimal invasiven Eingriff möglich, einen Teil des Trabekelmaschenwerkes (im Wesentlichen durch Skalpell und auch Hitze-Einwirkung) zu entfernen. Ein sehr vorteilhaftes chirugirsches Instrument für einen solchen Eingriff ist das Trabekom. Dies geschieht aus der Operationsrichtung O. Dabei können auch Teile der vorderen Wand vW des Schlemm'schen Kanals S entfernt werden. Die Entfernung geschieht dabei über einen begrenzten Winkelbereich, beispielsweise über 30°, 45° oder auch 90° sowie es in Fig. 4 angedeutet ist. (Die Winkel sind also auf des von dem Schlemm'schen Kanal als Zirkulargefäß abgedeckten Winkel von 360° bezogen.)

Diese Operationstechnik hinterlässt minimale Schäden am Auge und hat sich als sehr effektiv erwiesen. Allerdings ist die längerfristige Wirkung der Operation nicht immer ganz zufriedenstellend.

Im Zusammenhang mit der vorliegenden Erfindung wurde erkannt, dass der verbleibende Schlemm'sche Kanal seine Aufgabe nicht immer effizient wahrnehmen kann. Wie erkannt wurde, trägt dazu eine mangelhafte mechanische Stabilität des verbleibenden Schlemm'schen Kanals bei.

Das Implantat der vorliegenden Erfindung kann an der Stelle eingesetzt werden, an der Schlemm'scher Kanal entfernt wurde. Die Implantatlänge ist also auf die Länge des Schlemm'schen Kanals abzustimmen. Bei einem Erwachsenen hat der Schlemm'sche Kanal typischerweise einen Durchmesser von 12 bis 14 mm. Dementsprechend hat der Schlemm'sche Kanal typischerweise einen Umfang von 50 bis 75 mm haben. Wenn bei einem Schlemm'schen Kanal von 50 mm Umfang ein Winkelsegment von 30° entnommen wird, so würde eine Lücke von gut 4 mm bleiben. Das Implantat sollte dann eine Länge von über 4 mm haben. In der Regel ist es zweckmäßig, wenn für den beschriebenen Fall das Verbindungsstück eine Länge von genau 4 mm hat und die Stützbereiche eine Länge von etwa jeweils 1 mm haben. Diese Längenangaben sind nur ganz ungefähr und ein Operateur wird ein Implantat für die jeweilige Anwendung unter Berücksichtigung vieler Nebenbedingungen auswählen.

Indem der erste Stützbereich in einem ersten eröffneten Abschnitt des Schlemm'schen Kanals eingesetzt wird und der zweite Stützbereich gegenüberliegend in einem zweiten eröffneten verbleibenden Bereich des Schlemm'schen Kanals eingesetzt wird, wird eine starke mechanische Stabilisierung erreicht.

Dazu trägt ein distanzstabiles Verbindungsstück bei, welches die Position der entstandenen Endstücke des Schlemm'schen Kanals auf der Umfangslinie rund um die Hornhaut festlegt.

Nicht minder entscheidend ist die mechanische Stabilisierung der verbleibenden Endabschnitte des Schlemm'schen Kanals in Richtung des in Fig. 5 gezeigten Querschnittes.

Dieser Effekt ist noch einmal in Fig. 6 erkennbar. Der Stützbereich wird so in den Schlemm'schen Kanal S eingesetzt, dass dieser Druck, welcher innerhalb von der Darstellungsebene wirksam wird, besser widersteht.

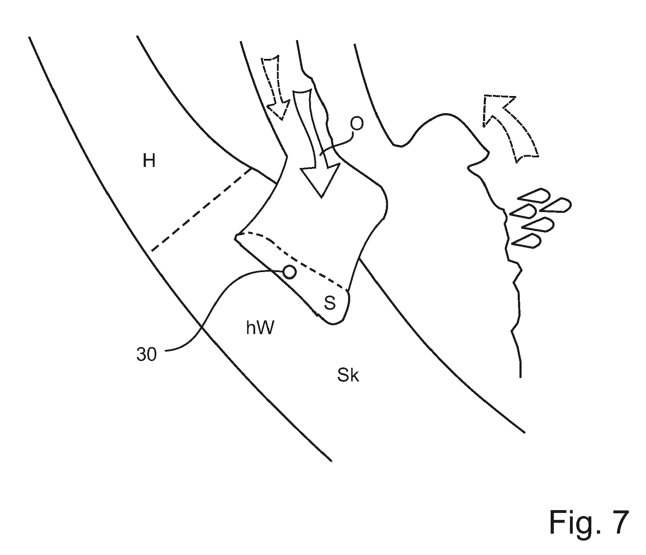

Fig. 7 zeigt mit einer weiteren Querschnittsansicht die Lage des Implantats. Wie in Fig. 3 angegeben, ist in dieser Ansicht nur eine Querschnittsfläche durch das Verbindungsstück 30 erkennbar.

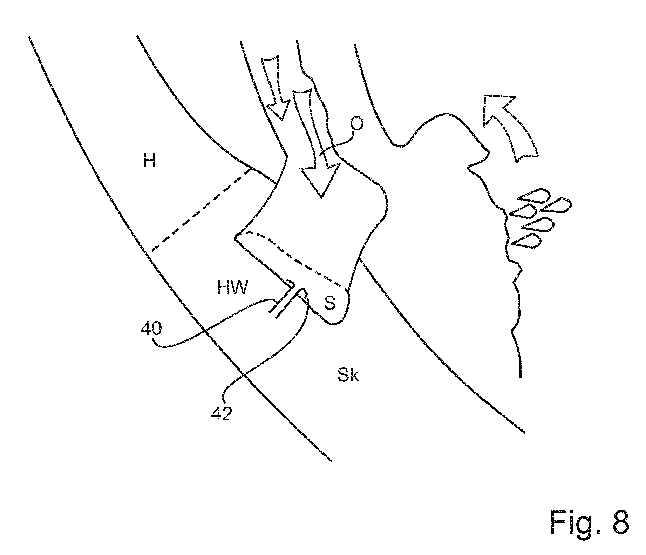

Fig. 8 zeigt eine weitere Querschnittsansicht. In dieser Querschnittsansicht erkennt man die Lage eines Tubus 40. Dieser Tubus wird in das Gewebe der hinteren Wand hW eingebracht. Vorteilhafterweise wird er genau in einen dort befindlichen Sammelkanal eingepasst. Der Tubusdurchmesser sollte dazu 20% bis 50% größer sein als der Durchmesser des Sammelkanals.

Vorteilhaft ist, wenn der Tubus über etwa 20% bis 30% seiner Länge aus dem Gewebe herausragt (also in den Bereich des eröffneten Schlemm'schen Kanals hinein). Dies vermeidet, dass Gewebe in den Tubus hineinwächst und diesen verschließt. Der Tubus 40 kann auch mit einem Rand 42 ausgerüstet werden, der ebenfalls hilft, Gewebe vom Tubuseingang fernzuhalten.

Es hat sich ebenfalls als vorteilhaft erwiesen, wenn die Tuben glatte Wände haben, da anderenfalls eine ungünstige Verwachsung mit Gewebe oder auch ein Zuwachsen des neu geschaffenen Flüssigkeitsableitweges beobachtet wurde.

Die Beschreibung hat auch deutlich gemacht, dass es sich bei dem erfindungsgemäßen Implantat nicht um einen Stent im üblichen Sinne handelt. Ein Stent im herkömmlichen Sinne dient dazu, ein Gefäß, beispielsweise ein Blutgefäß zu weiten, um so den Durchfluss zu verbessern. Das vorliegende Implantat überbrückt dagegen eine Lücke, welche durch Entfernen von Gefäßmaterial entsteht. Insofern geht seine Funktion über die eines Stents hinaus.

Im Rahmen der Erfindung wurde erkannt, dass sich bei Überbrückung eine solchen Lücke im Schlemm'schen Kanal ein wesentlicher zusätzlicher Effekt ergibt. In den Bereichen, in welchen Teile des Schlemm'schen Kanals entfernt wurden und eventuell auch eine Beschädigung des Gewebes durch mechanische Einwirkung oder durch Hitze nicht ganz ausgeschlossen werden kann, wird eine verbesserte Flüssigkeitsableitung erreicht. Flüssigkeit wird in die hintere Wand und damit in die Sklera Sk geleitet. Von dort die Flüssigkeit regulär abgeleitet werden. Dies wird bereits durch ein Implantat ohne Tuben, besonders aber durch eines mit Tuben erreicht.

Insgesamt wurde deutlich, wie mit dem erfindungsgemäßen Implantat die Wirkungen einer minimal invasiven Operation deutlich verbessert werden konnten. Diese Verbesserungen betreffen das Volumen des Augenwasserabflusses aber auch die zeitliche Stabilität der Wirkung.

### Bezugszeichenliste

- 10: Implantat
- 20: Brücke
- 22: erster Endpunkt
- 24: zweiter Endpunkt
- 26: erster Stützbereich
- 28: zweiter Stützbereich
- 30: Verbindungsstück
- 32: erste Spirale
- 34: zweite Spirale
- 36: erster Tubus
- 38: zweiter Tubus
- 40: dritter Tubus
- 42: Rand

- hA: hintere Augenkammer
- vA: vordere Augenkammer
- G: Glaskörper
- H: Hornhaut
- I: Iris
- K: konventionelles Operationsfeld
- L: Linse
- O: minimalinvasive Operationsrichtung
- T: Trabekelmaschenwerk
- Sk: Sklera
- S: Schlemm'scher Kanal
- vW: vordere Wand des Schlemm'schen Kanals
- hW: hintere Wand des Schlemm'schen Kanals
- L₁: erste Linie
- E₁: erste Ebene

## Patentansprüche

1. Implantat (10) für den Schlemm'schen Kanal, welches eine Brücke (20) mit einem ersten Endpunkt (22) und einem zweiten Endpunkt (24) und einem ersten Stützbereich (26), einem zweiten Stützbereich (28) und ein Verbindungsstück (30) umfasst, wobei sich die Brücke (20) entlang einer ersten Linie (L₁) vom ersten Endpunkt (22) über den ersten Stützbereich (26), das Verbindungsstück (30) und den zweiten Stützbereich (28) zum zweiten Endpunkt (24) erstreckt und wobei das Verbindungsstück (30) entlang der ersten Linie (L₁) im Durchschnitt eine erste Querschnittsfläche aufweist und der erste Stützbereich (26) nach der Implantation entlang der ersten Linie (L₁) im Durchschnitt eine zweite Querschnittsfläche aufweist, wobei die zweite Querschnittsfläche um mindestens 50% größer ist als die erste Querschnittsfläche, **dadurch gekennzeichnet dass** zumindest das Verbindungsstück (30) massiv ist.

2. Implantat (10) nach Anspruch 1, bei dem der zweite Stützbereich (28) nach der Implantation entlang der ersten Linie (L₁) im Durchschnitt eine dritte Querschnittsfläche aufweist und die dritte Querschnittsfläche um mindestens 50% größer ist als die erste Querschnittsfläche.

3. Implantat (10) nach einem der vorhergehenden Ansprüche, bei dem sich der erste Stützbereich (26) zur Implantation in eine Stellung überführen lässt, in der er entlang der ersten Linie (L₁) im Durchschnitt eine vierte Querschnittsfläche aufweist, welche um nicht mehr als 40% größer ist als die erste Querschnittsfläche.

4. Implantat (10) nach einem der vorhergehenden Ansprüche, bei dem sich der zweite Stützbereich (28) zur Implantation in eine Stellung überführen lässt, in der er entlang der ersten Linie (L₁) im Durchschnitt eine fünfte Querschnittsfläche aufweist, welche um nicht mehr als 40% größer ist als die erste Querschnittsfläche.

5. Implantat (10) nach einem der vorhergehenden Ansprüche, bei dem der erste Stützbereich (26) und/oder der zweite Stützbereich (28) nach Implantation eine Spiral-Form annehmen.

6. Implantat (10) nach einem der vorhergehenden Ansprüche, bei dem der erste Stützbereich (26) und/oder der zweite Stützbereich (28) aus einer Formgedächtnislegierung gefertigt sind.

7. Implantat (10) nach einem der vorhergehenden Ansprüche, bei dem das Verbindungsstück (30) biegsam ist.

8. Implantat (10) nach einem der vorhergehenden Ansprüche, bei dem das Verbindungsstück (30) eine geringere Biegesteifigkeit hat als der erste Stützbereich (26).

9. Implantat (10) nach einem der vorhergehenden Ansprüche, bei dem das Verbindungsstück (30) distanzstabil ist.

10. Implantat (10) nach einem der vorhergehenden Ansprüche, bei dem die Länge Verbindungsstückes (30) seinen Durchmesser um einen Faktor von mindestens 10 übersteigt.

11. Implantat (10) nach einem der vorhergehenden Ansprüche, welches nach der Implantation eine Stellung einnimmt, bei der die erste Linie (L₁) gekrümmt ist und in einer ersten Ebene (E₁) verläuft und welches mindestens einen Tubus (36) aufweist, welcher eine Flüssigkeitsleitung durch die erste Ebene (E₁) ermöglicht und vorzugsweise im Wesentlichen senkrecht zur ersten Ebene (E₁) orientiert ist.

12. Implantat (10) nach dem vorhergehenden Anspruch, bei welchem der mindestens eine Tubus (36) einen Durchmesser aufweist, welcher weniger als 10% der Länge des Verbindungsstücks (30) ausmacht.

## Claims

1. An implant (10) for the Schlemm's canal, said implant comprising a bridge (20) with a first end point (22) and a second end point (24) and a first support region (26), a second support region (28) and a connecting piece (30), wherein the bridge (20) extends along a first line (L1) from the first end point (22) to the second end point (24) across the first support region (26), the connecting piece (30) and the second support region (28), and wherein the connecting piece (30) has a first cross-sectional area on average along the first line (L1) and the first support region (26) following implantation has a second cross-sectional area on average along the first line (L1), wherein the second cross-sectional area is at least 50 % larger than the first cross-sectional area **characterised in that** at least the connecting piece (30) is solid.

2. The implant (10) according to Claim 1, wherein the second support region (28) following implantation has a third cross-sectional area on average along the first line (L1), and the third cross-sectional area is at least 50 % larger than the first cross-sectional area.

3. The implant (10) according to one of the preceding claims, wherein the first support region (26) for implantation can be transferred into a position in which said implant along the first line (L1) has a fourth cross-sectional area on average, which is no more than 40 % larger than the first cross-sectional area.

4. The implant (10) according to one of the preceding claims, wherein the second support region (28) for implantation can be transferred into a position in which it has along the first line (L1) a fifth cross-sectional area on average, which is no more than 40 % larger than the first cross-sectional area.

5. The implant (10) according to one of the preceding claims, wherein the first support region (26) and/or the second support region (28) adopt/adopts a spiral form following implantation.

6. The implant (10) according to one of the preceding claims, wherein the first support region (26) and/or the second support region (28) is/are fabricated from a shape-memory alloy.

7. The implant (10) according to one of the preceding claims, wherein the connecting piece (30) is flexible.

8. The implant (10) according to one of the preceding claims, wherein the connecting piece (30) has a more lower flexural rigidity compared to the first support region (26).

9. The implant (10) according to one of the preceding claims, wherein the connecting piece (30) is distance-stable.

10. The implant (10) according to one of the preceding claims, wherein the length of the connecting piece (30) exceeds the diameter thereof by a factor of at least 10.

11. The implant (10) according to one of the preceding claims, said implant after implantation adopting a position in which the first line (L1) is curved and runs in a first plane (E1) and has at least one tube (36), which enables fluid conduction through the first plane (E1) and is preferably oriented substantially perpendicularly to the first plane (E1).

12. The implant (10) according to the preceding claim, wherein the at least one tube (36) has a diameter which makes up less than 10 % of the length of the connecting piece (30).

## Revendications

1. Implant (10) pour le canal de Schlemm, ledit implant comprenant un pont (20) avec un premier point d'extrémité (22) et un second point d'extrémité (24) et une première région de support (26), une seconde région de support (28) et une pièce de liaison (30), dans lequel le pont (20) se prolonge le long d'une première ligne (L1) depuis le premier point d'extrémité (22) vers le second point d'extrémité (24) à travers la première région de support (26), la pièce de liaison (30) et la seconde région de support (28), et où la pièce de liaison (30) a une première surface transversale en moyenne le long de la première ligne (L1) et la première région de support (26) après l'implantation a une seconde surface transversale le long de la première ligne (L1), où la seconde surface transversale est d'au moins 50 % plus grande que la première surface transversale, **caractérisé en ce qu'**au moins la pièce de liaison (30) est solide.

2. Implant (10) selon la revendication 1, dans lequel la seconde région de support (28) après l'implantation a une troisième surface transversale en moyenne le long de la première ligne (L1), et la troisième surface transversale est d'au moins 50% plus grande que la première surface transversale.

3. Implant (10) selon l'une quelconque des revendications précédentes, dans lequel la première région de support (26) pour l'implantation peut être transférée dans une position dans laquelle ledit implant le long de la première ligne (L1) a une quatrième surface transversale en moyenne, qui ne dépasse pas de 40 % la première surface transversale.

4. Implant (10) selon l'une quelconque des revendications précédentes, dans lequel la seconde région de support (28) pour l'implantation peut être transférée dans une position dans laquelle elle a le long de la première ligne line (L1) une cinquième surface transversale en moyenne, qui ne dépasse pas de 40 % la première surface transversale.

5. Implant (10) selon l'une quelconque des revendications précédentes, dans lequel la première région de support (26) et/ou la seconde région de support (28) prend / prennent une forme en spirale après l'implantation.

6. Implant (10) selon l'une quelconque des revendications précédentes, dans lequel la première région de support (26) et/ou la seconde région de support (28) est fabriquée / sont fabriquées à partir d'un alliage à mémoire de forme.

7. Implant (10) selon l'une quelconque des revendications précédentes, dans lequel la pièce de liaison (30) est souple.

8. Implant (10) selon l'une quelconque des revendications précédentes, dans lequel la pièce de liaison (30) a une rigidité à la flexion plus petite comparativement à la première région de support (26).

9. Implant (10) selon l'une quelconque des revendications précédentes, dans lequel la pièce de liaison (30) est à une distance stationnaire.

10. Implant (10) selon l'une quelconque des revendications précédentes, dans lequel la longueur de la pièce de liaison (30) dépasse le diamètre de celle-ci d'un facteur d'au moins 10.

11. Implant (10) selon l'une quelconque des revendications précédentes, ledit implant après implantation adoptant une position dans laquelle la première ligne (L1) est courbe et s'étend dans un premier plan (E1) et a au moins un tube (36), qui permet un transport de fluide à travers le premier plan (E1) et est de préférence orienté substantiellement perpendiculaire par rapport au premier plan (E1).

12. Implant (10) selon la revendication précédente, dans lequel l'au moins un tube (36) a un diamètre qui fait jusqu'à moins de 10 % de la longueur de la pièce de liaison (30).
